# EUROPÄISCHE PATENTSCHRIFT

(11) **EP 1 797 889 B1**
(45) Veröffentlichungstag und Bekanntmachung des Hinweises auf die Patenterteilung: **10.02.2010**
(21) Anmeldenummer: 06025088.3
(22) Anmeldetag: 05.12.2006
(51) Int. Cl.: A61K 33/44, A61P 1/00, A61P 1/12

(54) **Aktivkohle für die medizinische Verwendung**
Activated carbon for medical use
Charbone activé à usage médical

(30) Priorität: 19.12.2005 DE 102005061120; 22.12.2005 DE 102005062160
(43) Veröffentlichungstag der Anmeldung: 20.06.2007
(73) Patentinhaber: Blücher GmbH, 40699 Erkrath (DE)
(72) Erfinder: von Blücher, Hasso, 40699 Erkrath (DE); Klemund, Michael, 40239 Düsseldorf (DE); Böhm, Oliver, 61348 Homburg (DE)
(74) Vertreter: Gesthuysen, von Rohr & Eggert

(56) Entgegenhaltungen:
- EP-A- 1 525 886
- EP-B1- 1 440 692

## Beschreibung

Die vorliegende Erfindung betrifft das Gebiet der Aktivkohle für die medizinische Verwendung. Insbesondere betrifft die vorliegende Erfindung die Verwendung einer teilchenförmigen Aktivkohle mit hoher Mikroporosität für die humanmedizinische oder veterinärmedizinische Anwendung. Des weiteren betrifft die vorliegende Erfindung eine pharmazeutische Zusammensetzung, welche eine derartige teilchenförmige Aktivkohle enthält und sich für die humanmedizinische oder veterinärmedizinische Anwendung eignet.

Aktivkohle, insbesondere in Form sogenannter medizinischer Kohle, wird im Bereich der Medizin insbesondere zur Adsorption von Giftstoffen, Bakterien, Gasen etc. im Magen/Darm-Trakt verabreicht. Haupteinsatzgebiet sind Durchfallerkrankungen (Diarrhoe) sowie Vergiftungen. Im Falle der Gabe von Aktivkohle bei Vergiftungen verhindert die Aktivkohle bei Vergiftungen mit Stoffen, die einem enterohepatischen Kreislauf unterliegen (z. B. Carbamazepin, Phenobarbital, Phenylbutazon, Theophyllin etc.), die Resorption und führt zu einer Beschleunigung deren Elimination.

Für weitere Einzelheiten zur medizinischen Anwendung von Aktivkohle kann beispielsweise verwiesen werden auf Römpp Chemielexikon, 10. Auflage, Georg Thieme Verlag, Stuttgart/New York, Stichwörter: "Aktivkohle" und "Medizinische Kohle", sowie auf Pschyrembel Medizinisches Wörterbuch, 257. Auflage, 1993, Nikol Verlagsgesellschaft mbH Hamburg, Stichwort: "Aktivkohle", und die dort jeweils referierte Literatur.

Für die medizinischen Anwendungsgebiete akuter Durchfall (Diarrhoe), zur Verhinderung der Resorption bei oralen Vergiftungen sowie zur Beschleunigung der Ausscheidung bei Vergiftungen mit Stoffen, die einem enterohepatischen Kreislauf unterliegen, sind handelsübliche Präparate auf der Basis pulverförmiger medizinischer Aktivkohle in Form sogenannter Kompretten verfügbar. Die zur Verfügung stehende pulverförmige Aktivkohle bietet jedoch für die vorgenannten Anwendungsgebiete nicht immer optimale Leistungseigenschaften.

Des weiteren wurde Aktivkohle in Form kugelförmiger Partikel mit Größen von 0,05 bis 2 mm bereits für andere medizinische Anwendungen vorgeschlagen, nämlich für die Behandlung von Hämorrhoidalerkrankungen (EP 0 688 566 B1), zur Behandlung entzündlicher Erkrankungen des Darms wie z. B. Morbus Crohn oder Colitis ulcerosa (EP 0 688 567 B1) sowie zur Behandlung stomaperiphärer Entzündungen (EP 0 688 568 B1). Jedoch erfüllt auch die in den vorgenannten Druckschriften beschriebene kugelförmige Aktivkohle nicht immer die Anforderungen, die an eine medizinische Aktivkohle gestellt wird.

Auch die aus Phenolharzkügelchen durch Carbonisierung und nachfolgende Aktivierung hergestellte Aktivkohle gemäß der EP 1 440 692 B1 erfüllt nicht stets das gewünschte Anforderungsprofil, welches an eine medizinische Aktivkohle gestellt wird.

Es ist daher eine Aufgabe der vorliegenden Erfindung, eine für die Verwendung im Bereich der Human- oder Veterinärmedizin verwendbare Aktivkohle aufzufinden bzw. bereitzustellen, welche die zuvor geschilderten Nachteile des Standes der Technik zumindest weitgehend vermeidet oder aber wenigstens abschwächt.

Insbesondere liegt eine Aufgabe der vorliegenden Erfindung in der Auffindung bzw. Bereitstellung einer Aktivkohle bzw. einer medizinischen Kohle, welche gute Anwendungseigenschaften im Bereich der Medizin, insbesondere Human- oder Veterinärmedizin, aufweist.

Die Anmelderin hat nun überraschender Weise gefunden, daß das zuvor geschilderte Problem durch eine Aktivkohle mit großem Mikroporenvolumenanteil, bezogen auf das Gesamtporenvolumen der Aktivkohle, gelöst werden kann.

Gegenstand der vorliegenden Erfindung ist die Verwendung einer teilchenförmigen Aktivkohle, insbesondere in Form von Aktivkohlekörnern, vorzugsweise Aktivkohlekügelchen, zur Herstellung eines Arzneimittels zur Behandlung von Erkrankungen des Magen/Darm-Traktes, wobei die eingesetzte Aktivkohle durch Carbonisierung und nachfolgende Aktivierung von Styrol/Divinylbenzol-Copolymeren erhältlich ist und wobei die eingesetzte Aktivkohle einen aus Mikroporen mit Porendurchmessern von ≤ 25 Å gebildeten Volumenanteil von mindestens 60 %, bezogen auf das Gesamtporenvolumen der Aktivkohle, bei einem Gesamtporenvolumen nach Gurvich von 0,50 bis 0,90 cm³/g aufweist.

Denn die Anmelderin hat überraschenderweise herausgefunden, daß teilchenförmige, insbesondere kornförmige, vorzugsweise kugelförmige Aktivkohle mit einem großen Mikroporenvolumenanteil eine besonders gute medizinische Aktivität besitzt, und dies für verschiedene Anwendungsbereiche der Medizin. Wenn im Rahmen der vorliegenden Erfindung der Bereich der Medizin angesprochen ist, so ist hiermit stets sowohl der Bereich der Humanmedizin als auch der Bereich der Veterinärmedizin zu verstehen.

Ohne sich auf eine bestimmte Theorie festlegen zu wollen, läßt sich die besondere Effektivität der erfindungsgemäß eingesetzten teilchenförmigen Aktivkohle mit einem großen Mikroporenvolumenanteil darauf zurückführen, daß eine solche Aktivkohle Giftstoffe, Mikroorganismen, wie Bakterien und Viren, schädliche Gase und dergleichen in besonders effizienter Weise adsorbieren kann, da die Mikroporen über die gesamte Porenwandung mit den Adsorbaten in Wechselwirkung treten können und diese besonders effektiv adsorbieren können.

Aufgrund dieser Eigenschaften ist die erfindungsgemäß eingesetzte Aktivkohle besonders wirksam, insbesondere im Vergleich zu marktüblichen Produkten, und muß folglich in nur geringeren Dosen eingesetzt werden als handelsübliche Aktivkohlepräparate.

Wenn im Rahmen der vorliegenden Erfindung von einem großen Volumenanteil an Mikroporen die Rede ist, so sind mit dem Begriff der Mikroporen solche Poren innerhalb der Aktivkohle gemeint, welche Porendurchmesser ≤ 25 Å (2,5 nm), insbesondere ≤ 20 Å (2,0 nm), aufweisen.

Die erfindungsgemäß verwendete Aktivkohle eignet sich für die Behandlung von Erkrankungen des Magen/Darm-Traktes bei Mensch und Tier bzw. zur Herstellung eines Arzneimittels zur Behandlung von Erkrankungen des Magen/Darm-Traktes. Beispielsweise kann die erfindungsgemäß verwendete Aktivkohle zur Behandlung von entzündlichen Erkrankungen des Magen/Darm-Traktes, insbesondere bakteriellen oder viralen Erkrankungen des Magen/Darm-Traktes, eingesetzt werden. Beispielsweise kann die erfindungsgemäß eingesetzte Aktivkohle zur Behandlung von entzündlichen, insbesondere bakteriell oder viral bedingten Erkrankungen des Dünn- und/oder Dickdarms, insbesondere der Enterocolitis, eingesetzt werden. Weiterhin kann die erfindungsgemäß verwendete Aktivkohle zur Behandlung von Durchfallerkrankungen (Diarrhoe) eingesetzt werden. Schließlich kann die erfindungsgemäß eingesetzte Aktivkohle zur Behandlung von Vergiftungen, insbesondere Lebensmittelvergiftungen oder Vergiftungen nach peroraler Aufnahme toxischer Substanzen, eingesetzt werden. So kann die erfindungsgemäß eingesetzte Aktivkohle bei oralen Vergiftungen die Resorption der Gifte durch den Organismus verhindern und im Fall von Vergiftungen mit Stoffen, die einem enterohepatischen Kreislauf unterliegen (z. B. Carbamazepin, Phenobarbital, Phenylbutazon, Theophyllin etc.), deren Ausscheidung bzw. Eliminierung beschleunigen.

Hauptanwendungsgebiet der erfindungsgemäß eingesetzten Aktivkohle sind Durchfallerkrankungen (Diarrhoe) sowie Vergiftungen.

Zu Zwecken ihrer medizinischen Anwendung wird die erfindungsgemäß eingesetzte Aktivkohle üblicherweise peroral appliziert. Zu diesem Zweck kann die teilchenförmige, insbesondere kugelförmige Aktivkohle entweder als solche verabreicht werden oder aber in eine spezielle Verabreichungsform (z. B. Kapseln etc.) gebracht werden.

Die applizierten Mengen richten sich nach der Art der Indikation bzw. Anwendung: Während bei der Behandlung von Vergiftungen etwa 0,1 bis 5 g Aktivkohle/kg Körpergewicht, insbesondere 0,2 bis 2 g Aktivkohle/kg Körpergewicht, eingesetzt werden, werden bei den anderen vorgenannten Indikationen täglich insgesamt 100 mg bis 5.000 mg, insbesondere 200 bis 4.500 mg, vorzugsweise 500 bis 4.000 mg, peroral appliziert. Trotzdem kann es gegebenenfalls erforderlich sein, von den vorgenannten Mengen abzuweichen, und zwar in Abhängigkeit vom Körpergewicht bzw. von der Art der Applikation, vom individuellen Verhalten, von der Art der Formulierung und von dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muß. Üblicherweise ist es empfehlenswert, Applikationen über einen definierten Zeitraum zu verteilen, und zwar vorteilhafterweise z. B. in mehreren Einzelgaben.

Vorzugsweise besteht die erfindungsgemäß eingesetzte Aktivkohle aus diskreten Aktivkohlekörnern, vorzugsweise diskreten Aktivkohlekügelchen. Die Aktivkohlekörner, vorzugsweise Aktivkohlekügelchen, besitzen dabei mittlere Teilchendurchmesser im Bereich von 0,01 bis 2,0 mm, insbesondere 0,05 bis 1,0 mm, vorzugsweise 0,1 bis 1,0 mm. Besonders vorteilhaft wird eine Aktivkohle verwendet, die besonders mechanisch stabil ist und einen Berstdruck von mindestens 5 Newton, insbesondere einen Berstdruck im Bereich von 5 Newton bis 20 Newton, pro Aktivkohlekörnchen bzw. Aktivkohlekügelchen aufweist.

Erfindungsgemäß bevorzugt eingesetzte Aktivkohle besitzt eine Rohdichte im Bereich von 700 bis 975 g/cm³, insbesondere 750 bis 950 g/cm³, vorzugsweise 800 bis 900 g/cm³. Weiterhin besitzt sie eine Gesamtporosität von 40 bis 70 %, insbesondere 45 bis 65 %, vorzugsweise 50 bis 60 %.

Erfindungsgemäß bevorzugt eingesetzte Aktivkohle besitzt ein spezifisches Gesamtporenvolumen im Bereich von 0,1 bis 2,5 cm³/g, insbesondere 0,2 bis 2,0 cm³/g, vorzugsweise 0,3 bis 1,5 cm³/g, besonders bevorzugt 0,4 bis 1,0 cm³/g. Dabei beträgt vorteilhafterweise der Anteil an Poren mit Porendurchmessern ≤ 36 Å mindestens 65 %, insbesondere mindestens 70 %, vorzugsweise mindestens 75 %, und kann bis zu 95 %, insbesondere bis zu 90 %, betragen.

Für die medizinische Anwendung ist es besonders vorteilhaft, wenn die eingesetzte Aktivkohle eine spezifische Oberfläche (BET-Oberfläche) von mindestens 500 g/m², insbesondere mindestens 750 g/m², vorzugsweise mindestens 1.000 g/m², besonders bevorzugt mindestens 1.200 g/m², aufweist. Üblicherweise besitzt die eingesetzte Aktivkohle eine spezifische Oberfläche (BET-Oberfläche) im Bereich von 500 bis 2.500 g/m², insbesondere 750 bis 2.250 g/m², vorzugsweise 900 bis 2.000 g/m², besonders bevorzugt 1.000 bis 1.750 g/m². Die vorgenannten BET-Werte beziehen sich auf Poren mit Porendurchmessem bis 400 Å einschließlich. Zur BET-Methode kann beispielsweise verwiesen werden auf Römpp Chemielexikon, 10. Auflage, Georg Thieme Verlag, Stuttgart/New York, Stichwort: "BET-Methode", sowie die dort referierte Literatur, Winnacker-Küchler (3. Auflage), Band 7, Seiten 93 ff. sowie Z. Anal. Chem. 238, Seiten 187 bis 193 (1968).

Im allgemeinen besitzt die erfindungsgemäß eingesetzte Aktivkohle ein Adsorptionsvolumen V_{ads} von mindestens 250 cm³/g, insbesondere mindestens 300 cm³/g, vorzugsweise mindestens 350 cm³/g, besonders bevorzugt mindestens 400 cm³/g. Im allgemeinen liegt das Adsorptionsvolumen V_{ads} der eingesetzten Aktivkohle im Bereich von 250 bis 1.000 cm³/g, insbesondere 300 bis 900 cm³/g, vorzugsweise 350 bis 750 cm³/g. Die vorgenannten Werte beziehen sich auf eine Messung des Adsorptionsvolumens bei einem Partialdruck p/p₀ von 0,995 an Poren mit Porendurchmessern im Bereich bis 400 Å einschließlich.

Die eingesetzte Aktivkohle weist ein Gesamtporenvolumen nach Gurvich von mindestens 0,50 cm³/g, insbesondere mindestens 0,55 cm³/g, vorzugsweise mindestens 0,60 cm³/g, besonders bevorzugt mindestens 0,65 cm³/g, ganz besonders bevorzugt mindestens 0,70 cm³/g, auf. Im allgemeinen besitzt die eingesetzte Aktivkohle ein Gesamtporenvolumen nach Gurvich von 0,50 bis 0,90 cm³/g, insbesondere 0,55 bis 0,85 cm³/g, vorzugsweise 0,60 bis 0,80 cm³/g, besonders bevorzugt 0,65 bis 0,80 cm³/g, ganz besonders bevorzugt 0,70 bis 0,75 cm³/g. Die vorgenannten Werte beziehen sich auf die Bestimmung bei einem Partialdruck p/p₀ von 0,995 an Poren mit einem Porendurchmesser bis 400 Å einschließlich. Zu weiteren Einzelheiten bezüglich der Bestimmung des Gesamtporenvolumens nach Gurvich kann verwiesen werden auf L. Gurvich (1915), J. Phys. Chem. Soc. Russ. 47, 805, sowie auf S. Lowell et al., Characterization of Porous Solids and Powders: Surface Area Pore Size and Density, Kluwer Academic Publishers, Article Technology Series, Seiten 111 ff.

Alle nachfolgend angegebenen physikalisch-chemischen Daten der Aktivkohle beziehen sich gleichermaßen auf Messungen an Poren mit Porendurchmessern bis 400 Å.

Wie zuvor beschrieben, ist eine Besonderheit der erfindungsgemäß eingesetzten Aktivkohle in ihrem besonders hohen Mikroporenvolumenanteil zu sehen. Wie zuvor definiert, bezeichnet der Begriff der Mikroporen solche Poren mit Porendurchmessern von ≤ 25 Å, insbesondere ≤ 20 Å. Folglich bezeichnet der Begriff des Mikroporenvolumenanteils denjenigen Volumenanteil, der durch Poren mit Porendurchmessern von ≤ 25 Å, insbesondere ≤ 20 Å, bereitgestellt wird. Bei der erfindungsgemäß eingesetzten Aktivkohle liegt der Anteil des Mikroporenvolumens, bezogen auf das Gesamtporenvolumen der Aktivkohle, bei mindestens 60 %, insbesondere bei mindestens 65 %, bevorzugt bei mindestens 70 %. Im allgemeinen liegt der Mikroporenvolumenanteil der eingesetzten Aktivkohle, bezogen auf das Gesamtporenvolumen der Aktivkohle, im Bereich von 60 bis 95 %, insbesondere 65 bis 90 %, vorzugsweise 70 bis 85 %. Untersuchungen der Anmelderin haben gezeigt, daß eine derart mikroporöse Aktivkohle für die medizinische Anwendung überraschenderweise besonders geeignet ist, insbesondere eine besonders gute medizinische Aktivität besitzt.

Im allgemeinen besitzt die erfindungsgemäß eingesetzte Aktivkohle ein Mikroporenvolumen, insbesondere ein aus Poren mit Porendurchmessern von ≤ 25 Å, vorzugsweise ≤ 20 Å, gebildetes Mikroporenvolumen nach Carbon Black von mindestens 0,40 cm³/g, insbesondere mindestens 0,45 cm³/g, vorzugsweise mindestens 0,50 cm³/g. Im allgemeinen liegt das Mikroporenvolumen der eingesetzten Aktivkohle (d. h. das aus Poren mit Porendurchmessern von ≤ 25 Å, vorzugsweise ≤ 20 Å gebildete Mikroporenvolumen) nach Carbon Black im Bereich von 0,40 bis 0,80 cm³/g, insbesondere 0,45 bis 0,75 cm³/g, vorzugsweise 0,50 bis 0,6 cm³/g. Zu weiteren Einzelheiten der Bestimmung der Porenoberfläche nach Carbon Black kann beispielsweise verwiesen werden auf R. W. Magee, Evaluation of the External Surface Area of Carbon Black by Nitrogen Adsorption, Presented at the Meeting of the Rubber Division of the American Chem. Soc., Oktober 1994, z. B. referiert in: Quantachrome Instruments, AUTOSORB-1, AS1 Win Version 1.50, Operating Manual, P/N 05061, Quantachrome Instruments 2004, Florida, USA, Seiten 71 ff.

Neben einem besonders hohen Mikroporenvolumenanteil besitzt die erfindungsgemäß eingesetzte Aktivkohle auch einen besonders hohen Mikroporenoberflächenanteil in bezug auf die Porengesamtoberfläche. Im allgemeinen besitzt die erfindungsgemäß eingesetzte Aktivkohle, bezogen auf die spezifische Gesamtoberfläche (BET) der Aktivkohle, einen spezifischen Mikroporenoberflächenanteil (d. h. einen aus Poren mit Porendurchmessern von ≤ 25 Å, vorzugsweise ≤ 20 Å, gebildeten spezifischen Mikroporenoberflächenanteil von mindestens 70 %, insbesondere mindestens 75 %, bevorzugt mindestens 80 %, ganz besonders bevorzugt mindestens 85 %. Im allgemeinen liegt der spezifische Mikroporenoberflächenanteil, bezogen auf die spezifische Gesamtoberfläche (BET), im Bereich von 70 bis 95 %, vorzugsweise 75 bis 95 %, ganz besonders bevorzugt 80 bis 90 %.

Im allgemeinen besitzt die erfindungsgemäß eingesetzte Aktivkohle eine Mikroporenoberfläche nach Carbon Black (d. h. eine aus Poren mit Porendurchmesser von ≤ 25 Å, vorzugsweise ≤ 20 Å, gebildete Mikroporenoberfläche nach Carbon Black) von mindestens 400 g/m², insbesondere mindestens 800 g/m², vorzugsweise mindestens 1.000 g/m², besonders bevorzugt mindestens 1.200 g/m². Im allgemeinen liegt die Mikroporenoberfläche nach Carbon Black im Bereich von 400 bis 1.750 g/m², insbesondere 800 bis 1.500 g/m², vorzugsweise 1.000 bis 1.400 g/m², besonders bevorzugt 1.100 bis 1.300 g/m².

Infolge ihrer hohen Mikroporosität besitzt die erfindungsgemäß eingesetzte Aktivkohle vorzugsweise einen mittleren Porendurchmesser (Durchschnittsporendurchmesser) von höchstens 35 Å, vorzugsweise höchstens 30 Å, besonders bevorzugt höchstens 25 Å. Im allgemeinen liegt der mittlere Porendurchmesser (Durchschnittsporendurchmesser) der erfindungsgemäß eingesetzten Aktivkohle im Bereich von 15 bis 35 Å, insbesondere 15 bis 30 Å, vorzugsweise 20 bis 25 Å.

Die erfindungsgemäß eingesetzte Aktivkohle ist durch Carbonisierung und nachfolgende Aktivierung von Styrol/Divinylbenzol-Copolymeren, insbesondere divinylbenzolvernetzten Polystyrolen, vorzugsweise in Kornform, besonders bevorzugt in Kugelform, erhältlich; insbesondere bei einem Divinylbenzolgehalt der als Ausgangsmaterialien für die Aktivkohleherstellung eingesetzten Styrol/Divinylbenzol-Copolymeren im Bereich von 1 bis 15 Gew.-%, vorzugsweise 2 bis 10 Gew.-%, bezogen auf die Styrol/Divinylbenzol-Copolymere, werden gute Anwendungsergebnisse erhalten. Beispielsweise ist eine derartige Aktivkohle einer ausgehend von Phenolharzkügelchen hergestellten Aktivkohle (vgl. z. B. EP 1 440 692 B1) in ihrer Wirkung überlegen.

Erfindungsgemäß einsetzbare Aktivkohle, welche die zuvor genannten Anforderungen bzw. Spezifikationen mit den vorgenannten physikalisch-chemischen Anforderungen erfüllt, wird beispielsweise von den Firmen Blücher GmbH, Erkrath, Deutschland, sowie Adsor-Tech GmbH, Premnitz, Deutschland, vertrieben.

Eine derartige Aktivkohle wird durch unter an sich bekannten Bedingungen durchgeführte Carbonisierung und nachfolgende Aktivierung geeigneter mikroporöser Ausgangsmaterialien, vorzugsweise auf der Basis divinylbenzolvemetzter Styrolharzkügelchen, hergestellt. Dies ist dem Fachmann geläufig, so daß diesbezüglich keine weiteren Einzelheiten aufgeführt zu werden brauchen.

Das erfindungsgemäß hergestellte Arzneimittel umfaßt jede Art von Arzneimittel bzw. Pharmaka als solche, aber auch Medizinprodukte, homöopathische Mittel, Nahrungsergänzungsmittel etc.

Neben der Aktivkohle kann das erfindungsgemäß hergestellte Arzneimittel außerdem einen pharmakologisch unbedenklichen Träger oder Exzipienten und/oder pharmakologisch unbedenkliche Hilfs- und/oder Trägerstoffe umfassen.

Das erfindungsgemäß hergestellte Arzneimittel liegt in Form einer peroral verabreichbaren Applikationsform vor, insbesondere in Form von Kapseln, Tabletten, Preßlingen, Kompretten, Pillen etc. Im allgemeinen enthält die pharmazeutische Zusammensetzung die Aktivkohle je Applikationseinheit, insbesondere je Tablette, Kapsel, Komprette, Preßling, Pille oder dergleichen, in Mengen von 100 mg bis 1.000 mg.

Die vorliegende Erfindung wird anhand des nachfolgenden Ausführungsbeispiels veranschaulicht, welches die vorliegende Erfindung jedoch keinesfalls beschränken soll.

**Ausführungsbeispiel:**
Dreißig Patienten mit akuten Durchfallerkrankungen (Diarrhoe) infolge eines Magen/Darm-Infektes wurden mit Aktivkohle behandelt, wobei zehn Patienten eine erfindungsgemäß eingesetzte Aktivkohle erhielten, während von den übrigen zwanzig Probanden zehn Probanden eine handelsübliche medizinische Aktivkohle auf Basis von Aktivkohlepulver in Form von Kompretten (Vergleich I) erhielten und den verbleibenden zehn Probanden eine kugelförmige Aktivkohle, welche gemäß der EP 1 440 692 B1 (Beispiel 1, mittlerer Teilchendurchmesser von ca. 0,1 mm) aus Phenolharzkügelchen hergestellt war , verabreicht wurde (Vergleich II).
Die erfindungsgemäß eingesetzte Aktivkohle, welche als solche in Form von Aktivkohlekügelchen eingesetzt wurde, wurde von der Adsor-Tech GmbH, Premnitz, Deutschland, bezogen und ist durch Carbonisierung und nachfolgende Aktivierung von kugelförmigen Styrol/Divinylbenzol-Copolymerpartikeln (Divinylbenzolgehalt von ca. 5 Gew.-%, bezogen auf die Styrol/Divinylbenzol-Copolymere) hergestellt; die erfindungsgemäß eingesetzte Aktivkohle weist die folgenden Eigenschaften auf:
   - Aktivkohlekügelchen mit mittleren Teilchendurchmesser von ca. 0,4 bis 0,6 mm
   - Berstdruck pro Aktivkohlekügelchen: > 5 Newton
   - BET-Gesamtoberfläche: ca. 1.400 m²/g
   - Adsorptionsvolumen V_{ads} (p/p₀ = 0,995): ca. 470 cm³/g
   - Gesamtporenvolumen nach Gurvich: ca. 0,72 cm³/g
   - Mikroporenvolumenanteil ≤ 20 Å, bezogen auf Gesamtporenvolumen bis 400 Å: ca. 70 %
   - Mikroporenvolumen ≤ 20 Å nach Carbon Black: ca. 0,51 cm³/g
   - Mikroporenoberflächenanteil, bezogen auf BET-Gesamtoberfläche: ca. 89 %
   - Mikroporenoberfläche ≤ 20 Å nach Carbon Black: ca. 1.250 m²/g
   - Mittlerer Porendurchmesser (Durchschnittsporendurchmesser): ca. 21 Å
   - Rohdichte: ca. 0,87 bis 0,89 g/cm³
   - Spezifisches Gesamtporenvolumen: ca. 0,64 bis 0,66 cm³/g
   - Spezifisches Porenvolumen ≤ 36 Å: ca. 0,50 bis 0,56 cm³/g

Bei den zehn Patienten, welche die handelsübliche medizinische Aktivkohle auf Basis eines Aktivkohlepulvers in Form von Aktivkohlekompretten (Vergleich I) erhielten, mußte die Therapie fünf Tage lang mit Tagesgesamtdosen von ca. 4.000 mg/diem durchgeführt werden, bis ein Therapieerfolg eintrat.

Auch bei den zehn Patienten, denen die kugelförmige Aktivkohle, welche gemäß der EP 1 440 692 B1 aus Phenolharzkügelchen hergestellt war, verabreicht wurde (Vergleich II), mußte die Therapie fünf Tage lang mit Tagesgesamtdosen von ca. 4.000 mg/diem durchgeführt werden, bis ein Therapieerfolg eintrat.

Dagegen war bei der erfindungsgemäß verwendeten mikroporösen Aktivkohle in Form von Aktivkohlekügelchen der zuvor beschriebenen Art auf Basis von Styrol/Divinylbenzol-Copolymeren (Fa. Adsor-Tech) die Therapie bei den zehn mit der erfindungsgemäß eingesetzten Aktivkohle behandelten Patienten bereits nach drei Tagen abgeschlossen, und dies bei einer Gesamttagesdosis an Aktivkohle von nur ca. 2.000 mg/diem.

Die voranstehenden Untersuchungen zeigen die verbesserten Effizienz der erfindungsgemäß eingesetzten Aktivkohle auf Basis mikroporöser Aktivkohlekügelchen auf Basis von Styrol/Divinylbenzol-Copolymeren sowohl im Vergleich zur handelsüblicher medizinischer Aktivkohle als auch im Vergleich zu phenolharzbasierter Aktivkohle.

Die vorliegenden Untersuchungen belegen die verbesserte Wirksamkeit von teilchenförmiger, insbesondere korn- bzw. kugelförmiger Aktivkohle mit hoher Mikroporosität, d. h. großem Mikroporenvolumen- sowie großem Mikroporenoberflächenanteil, auf Basis von Styrol/Divinylbenzol-Copolymeren (d h. die Aktivkohle ist durch Carbonisierung und nachfolgende Aktivierung dieser Styrol/Divinylbenzol-Copolymeren erhalten).

## Patentansprüche

1. Verwendung einer teilchenförmigen Aktivkohle, insbesondere in Form von Aktivkohlekörnern, vorzugsweise Aktivkohlekügelchen, zur Herstellung eines Arzneimittels zur Behandlung von Erkrankungen des Magen/Dann-Traktes, wobei die eingesetzte Aktivkohle durch Carbonisierung und nachfolgende Aktivierung von Styrol/Divinylbenzol-Copolymeren erhältlich ist und wobei die eingesetzte Aktivkohle einen aus Mikroporen mit Porendurchmessern von ≤ 25 Å gebildeten Volumenanteil von mindestens 60 %, bezogen auf das Gesamtporenvolumen der Aktivkohle, bei einem Gesamtporenvolumen nach Gurvich von 0,50 bis 0,90 cm³/g aufweist.

2. Verwendung nach Anspruch 1 zur Herstellung eines Arzneimittels zur Anwendung an einem Menschen oder an einem Tier.

3. Verwendung nach Anspruch 1 oder 2 zur Herstellung eines Arzneimittels zur Behandlung von entzündlichen Erkrankungen des Magen/Darm-Traktes .

4. Verwendung nach einem oder mehreren der vorangehenden Ansprüche zur Herstellung eines Arzneimittels zur Behandlung von entzündlichen, insbesondere bakteriell oder viral bedingten Erkrankungen des Dünn- und/oder Dickdarms, insbesondere Enterocolitis.

5. Verwendung nach einem oder mehreren der vorangehenden Ansprüche zur Herstellung eines Arzneimittels zur Behandlung von Durchfallerkrankungen (Diarrhoe).

6. Verwendung nach einem oder mehreren der vorangehenden Ansprüche zur Herstellung eines Arzneimittels zur Behandlung von Vergiftungen, insbesondere Lebensmittelvergiftungen oder Vergiftungen nach peroraler Aufnahme toxischer Substanzen.

7. Verwendung nach einem oder mehreren der vorangehenden Ansprüche, **dadurch gekennzeichnet, daß** die eingesetzte Aktivkohle diskrete Aktivkohlekömer, vorzugsweise Aktivkohlektigelchen, umfaßt oder hieraus besteht, insbesondere wobei die Aktivkohlekömer, vorzugsweise Aktivkohlekügelchen, einen Berstdruck von mindestens 5 Newton, insbesondere im Bereich von 5 Newton bis 20 Newton, pro Körnchen bzw. Kügelchen aufweisen, und/oder daß die eingesetzte Aktivkohle eine kugelförmige Aktivkohle ist und/oder daß die eingesetzte Aktivkohle Aktivkohlekörner, vorzugsweise Aktivkohlekügelchen, mit mittleren Teilchendurchmessern im Bereich von 0,01 bis 2,0 mm, insbesondere 0,05 bis 1,0 mm, vorzugsweise 0,1 bis 1,0 mm, aufweist.

8. Verwendung nach einem oder mehreren der vorangehenden Ansprüche, **dadurch gekennzeichnet, daß** die eingesetzte Aktivkohle erhältlich ist durch Carbonisierung und nachfolgende Aktivierung von Styrol/Divinylbenzol-Copolymeren in Kornform, besonders bevorzugt in Kugelform, und/oder daß der Divinylbenzolgehalt der Styrol/Divinylbenzol-Copolymeren im Bereich von 1 bis 15 Gew.-%, vorzugsweise 2 bis 10 Gew.-%, bezogen auf die Styrol/Divinylbenzol-Copolymere, beträgt.

9. Verwendung nach einem oder mehreren der vorangehenden Ansprüche, **dadurch gekennzeichnet, daß** die eingesetzte Aktivkohle erhältlich ist durch Carbonisierung und nachfolgende Aktivierung von divinylbenzolvernetzten Polystyrolen.

10. Verwendung nach einem oder mehreren der vorangehenden Ansprüche, **dadurch gekennzeichnet, daß** die eingesetzte Aktivkohle eine spezifische Oberfläche (BET-Oberfläche) von mindestens 500 m²/g, insbesondere mindestens 750 m²/g, vorzugsweise mindestens 1.000 m²/g, besonders bevorzugt mindestens 1.200 m²/g, aufweist und/oder daß die eingesetzte Aktivkohle eine spezifische Oberfläche (BET-Oberfläche) von 500 bis 2.500 m²/g, insbesondere 750 bis 2.250 m²/g, vorzugsweise 900 bis 2.000 m²/g, besonders bevorzugt 1.000 bis 1.750 m²/g, aufweist.

11. Verwendung nach einem oder mehreren der vorangehenden Ansprüche, **dadurch gekennzeichnet, daß** die eingesetzte Aktivkohle ein Adsorptionsvolumen V_{ads} von mindestens 250 cm³/g, insbesondere mindestens 300 cm³/g, vorzugsweise mindestens 350 cm³/g, besonders bevorzugt mindestens 400 cm³/g, aufweist und/oder daß die eingesetzte Aktivkohle ein Adsorptionsvolumen V_{ads} von 250 bis 1.000 cm³/g, insbesondere 300 bis 900 cm³/g, vorzugsweise 350 bis 750 cm³/g, aufweist und/oder daß die eingesetzte Aktivkohle ein Gesamtporenvolumen nach Gurvich von 0,55 bis 0,85 cm³/g, vorzugsweise 0,60 bis 0,80 cm³/g, besonders bevorzugt 0,65 bis 0,80 cm³/g, ganz besonders bevorzugt 0,70 bis 0,75 cm³/g, aufweist.

12. Verwendung nach einem oder mehreren der vorangehenden Ansprüche, **dadurch gekennzeichnet, daß** die eingesetzte Aktivkohle, bezogen auf das Gesamtporenvolumen, einen aus Mikroporen mit Porendurchmessern von ≤ 25 Å gebildeten Volumenanteil von mindestens 65 %, bevorzugt mindestens 70 %, aufweist.

13. Verwendung nach einem oder mehreren der vorangehenden Ansprüche, **dadurch gekennzeichnet, daß** die eingesetzte Aktivkohle, bezogen auf das Gesamtporenvolumen, einen aus Mikroporen mit Porendurchmessern von ≤ 20 Å gebildeten Porenvolumenanteil von mindestens 60 %, insbesondere mindestens 65 %, bevorzugt mindestens 70 %, aufweist und/oder daß die eingesetzte Aktivkohle, bezogen auf das Gesamtporenvolumen, einen aus Poren mit Porendurchmessern von ≤ 25 Å, insbesondere ≤ 20 Å, gebildeten Mikroporenvolumenanteil von 60 bis 95 %, insbesondere 65 bis 90 %, vorzugsweise 70 bis 85 %, aufweist.

14. Verwendung nach einem oder mehreren der vorangehenden Ansprüche, **dadurch gekennzeichnet, daß** die eingesetzte Aktivkohle ein aus Mikroporen mit Porendurchmessern von ≤ 25 Å, vorzugsweise ≤ 20 Å, gebildetes Porenvolumen nach Carbon Black von mindestens 0,40 cm³/g, insbesondere mindestens 0,45 cm³/g, vorzugsweise mindestens 0,50 cm³/g, aufweist und/oder daß die eingesetzte Aktivkohle ein aus Mikroporen mit Porendurchmessern von ≤ 25 Å, vorzugsweise ≤ 20 Å, gebildetes Porenvolumen nach Carbon Black von 0,40 bis 0,80 cm³/g, insbesondere 0,45 bis 0,75 cm³/g, vorzugsweise 0,50 bis 0,6 cm³/g, aufweist.

15. Verwendung nach einem oder mehreren der vorangehenden Ansprüche, **dadurch gekennzeichnet, daß** die eingesetzte Aktivkohle, bezogen auf die spezifische Gesamtoberfläche (BET) der Aktivkohle, einen aus Mikroporen mit Porendurchmessern von ≤ 25 Å, vorzugsweise ≤ 20 Å, gebildeten spezifischen Oberflächenanteil von mindestens 70 %, insbesondere mindestens 75 %, bevorzugt mindestens 80 %, ganz besonders bevorzugt mindestens 85 %, aufweist und/oder daß die eingesetzte Aktivkohle, bezogen auf die spezifische Gesalntoberfläche (BET) der Aktivkohle, einen aus Mikroporen mit Porendurchmessern von ≤ 25 Å, vorzugsweise ≤ 20 Å, gebildeten spezifischen Porenoberflächenanteil von 70 bis 95 %, insbesondere 75 bis 95 %, vorzugsweise 80 bis 90 %, aufweist.

16. Verwendung nach einem oder mehreren der vorangehenden Ansprüche, **dadurch gekennzeichnet, daß** die eingesetzte Aktivkohle eine aus Mikroporen mit Porendurchmessern von ≤ 25 Å, vorzugsweise ≤ 20 Å, gebildete Porenoberfläche nach Carbon Black von mindestens 400 m²/g, insbesondere mindestens 800 m²/g, vorzugsweise mindestens 1.000 m²/g, besonders bevorzugt mindestens 1.200 m²/g, aufweist und/oder daß die eingesetzte Aktivkohle nach Carbon Black eine aus Mikroporen mit Porendurchmessern von ≤ 25 Å, vorzugsweise s 20 Å, gebildete Porenoberfläche von 400 bis 1.750 m²/g, insbesondere 800 bis 1.500 m²/g, vorzugsweise 1.000 bis 1.400 m²/g, besonders bevorzugt 1.100 bis 1, 300m²/g, aufweist.

17. Verwendung nach einem oder mehreren der vorangehenden Ansprüche, **dadurch gekennzeichnet, daß** die eingesetzte Aktivkohle einen mittleren Porendurchmesser (Durchschnittsporendurchmesser) von höchstens 35 Å, vorzugsweise höchstens 30 Å, besonders bevorzugt höchstens 25 Å, aufweist und/oder daß die eingesetzte Aktivkohle einen mittleren Porendurchmesser (Durchschnittsporendurchmesser) im Bereich von 15 bis 35 Å, insbesondere 15 bis 30 Å, vorzugsweise 20 bis 25 Å, aufweist,

18. Verwendung nach einem oder mehreren der vorangehenden Ansprüche, **dadurch gekennzeichnet, daß** die eingesetzte Aktivkohle eine Rohdichte im Bereich von 700 bis 975 g/cm³, insbesondere 750 bis 950 g/cm³, vorzugsweise 800 bis 900 g/cm³, aufweist und/oder daß die eingesetzte Aktivkohle eine Gesamtporosität von 40 bis 70 %, insbesondere 45 bis 65 %, vorzugsweise 50 bis 60 %, aufweist und/oder daß die eingesetzte Aktivkohle eine spezifisches Gesamtporenvolumen im Bereich von 0,1 bis 2,5 cm³/g, insbesondere 0,2 bis 2,0 cm³/g, vorzugsweise 0,3 bis 1,5 cm³/g, besonders bevorzugt 0,4 bis 1,0 cm³/g, aufweist, insbesondere wobei der Anteil an Poren mit Porendurchmessern ≤ 36 Å mindestens 65 %, insbesondere mindestens 70 %, vorzugsweise mindestens 75 %, und bis zu 95 %, insbesondere bis zu 90 %, beträgt.

## Claims

1. Use of a particulate active carbon, in particular in the form of active carbon particles, preferably active carbon beads, for the production of a medicament for the treatment of complaints of the gastrointestinal tract, where the active carbon employed is obtainable by carbonization and subsequent activation of styrene/divinylbenzene copolymers and where the active carbon employed has a volume content formed from micropores having pore diameters of ≤ 25 Å, of at least 60 %, based on the total pore volume of the active carbon, with a total pore volume according to Gurrich of 0.50 to 0.90 cm³/g.

2. Use according to Claim 1 for the production of a medicament for use on a human or on an animal.

3. Use according to Claim 1 or 2 for the production of a medicament for treating inflammatory complaints of the gastrointestinal tract.

4. Use according to one or more of the preceding claims for the production of a medicament for treating inflammatory, in particular bacterially or virally related complaints of the small and/or large intestine, in particular enterocolitis.

5. Use according to one or more of the preceding claims for the production of a medicament for treating diarrhoeal complaints (diarrhoea).

6. Use according to one or more of the preceding claims for the production of a medicament for treating poisoning, in particular food poisoning or poisoning after peroral assimilation of toxic substances.

7. Use according to one or more of the preceding claims, **characterized in that** the active carbon employed comprises discrete active carbon particles, preferably active carbon beads, or consists thereof, in particular where the active carbon particles, preferably active carbon beads, have a bursting pressure of at least 5 Newtons, in particular in the range from 5 Newtons to 20 Newtons, per particle or bead, and/or **in that** the active carbon employed is a spherical active carbon and/or **in that** the active carbon employed contains active carbon particles, preferably active carbon beads, having median particle diameters in the range from 0.01 to 2.0 mm, in particular 0.05 to 1.0 mm, preferably 0.1 to 1.0 mm.

8. Use according to one or more of the preceding claims, **characterized in that** the active carbon employed is obtainable by carbonization and subsequent activation of styrene/divinylbenzene copolymers in particle form, particularly preferably in bead form, and/or **in that** the divinylbenzene content of the styrene/divinylbenzene copolymers is in the range from 1 to 15 % by weight, preferably 2 to 10 % by weight, based on the styrene/divinylbenzene copolymers.

9. Use according to one or more of the preceding claims, **characterized in that** the active carbon employed is obtainable by carbonization and subsequent activation of divinylbenzene-crosslinked polystyrenes.

10. Use according to one or more of the preceding claims, **characterized in that** the active carbon employed has a specific surface area (BET surface area) of at least 500 m²/g, in particular at least 750 m²/g, preferably at least 1000 m²/g, particularly preferably at least 1200 m²/g and/or **in that** the active carbon employed has a specific surface area (BET surface area) of 500 to 2500 m²/g, in particular 750 to 2250 m²/g, preferably 900 to 2000 m²/g, particularly preferably 1000 to 1750 m²/g.

11. Use according to one or more of the preceding claims, **characterized in that** the active carbon employed has an adsorption volume V_{ads} of at least 250 cm³/g, in particular at least 300 cm³/g, preferably at least 350 cm³/g, particularly preferably at least 400 cm³/g and/or **in that** the active carbon employed has an adsorption volume V_{ads} of 250 to 1000 cm³/g, in particular 300 to 900 cm³/g, preferably 350 to 750 cm³/g and/or **in that** the active carbon employed has a total pore volume according to Gurvich of 0.55 to 0.85 cm³/g, preferably 0.60 to 0.80 cm³/g, particularly preferably 0.65 to 0.80 cm³/g, very particularly preferably 0.70 to 0.75 cm³/g.

12. Use according to one or more of the preceding claims, **characterized in that** the active carbon employed, based on the total pore volume, has a volume content formed from micropores having pore diameters of ≤ 25 Å, of at least 65 %, preferably at least 70 %.

13. Use according to one or more of the preceding claims, **characterized in that** the active carbon employed, based on the total pore volume, has a pore volume content formed from micropores having pore diameters of < 20 Å, of at least 60 %, in particular at least 65 %, preferably at least 70 %, and/or **in that** the active carbon employed, based on the total pore volume, has a micropore volume content formed from pores having pore diameters of ≤ 25 Å, preferably ≤ 20 Å, of 60 to 95 %, in particular 65 to 90 %, preferably 70 to 85 %.

14. Use according to one or more of the preceding claims, **characterized in that** the active carbon employed has a pore volume formed from micropores having pore diameters of ≤ 25 Å, preferably ≤ 20 Å, according to Carbon Black of at least 0.40 cm³/g, in particular at least 0.45 cm³/g, preferably at least 0.50 cm³/g, and/or **in that** the active carbon employed has a pore volume formed from micropores having pore diameters of ≤ 25 Å, preferably ≤ 20 Å, according to Carbon Black of 0.40 to 0.80 cm³/g, in particular 0.45 to 0.75 cm³/g, preferably 0.50 to 0.6 cm³/g.

15. Use according to one or more of the preceding claims, **characterized in that** the active carbon employed, based on the specific total surface area (BET) of the active carbon, has a specific surface area content formed from micropores having pore diameters of ≤ 25 Å, preferably ≤ 20 Å, of at least 70 %, in particular at least 75 %, preferably at least 80 %, very particularly preferably at least 85 %, and/or **in that** the active carbon employed, based on the specific total surface area (BET) of the active carbon, has a specific pore surface area content formed from micropores having pore diameters of ≤ 25 Å, preferably ≤ 20 Å, of 70 to 95 %, in particular 75 to 95 %, preferably 80 to 90 %.

16. Use according to one or more of the preceding claims, **characterized in that** the active carbon employed has a pore surface area formed from micropores having pore diameters of ≤ 25 Å, preferably ≤ 20 Å, according to Carbon Black of at least 400 m²/g, in particular at least 800 m²/g, preferably at least 1000 m²/g, particularly preferably at least 1200 m²/g, and/or **in that** the active carbon employed according to Carbon Black has a pore surface area formed from micropores having pore diameters of ≤ 25 Å, preferably ≤ 20 Å, of 400 to 1750 m²/g, in particular 800 to 1500 m²/g, preferably 1000 to 1400 m²/g, particularly preferably 1100 to 1300 m²/g.

17. Use according to one or more of the preceding claims, **characterized in that** the active carbon employed has a median pore diameter (average pore diameter) of at most 35 Å, preferably at most 30 Å, particularly preferably at most 25 Å, and/or **in that** the active carbon employed has a median pore diameter (average pore diameter) in the range from 15 to 35 Å, in particular 15 to 30 Å, preferably 20 to 25 Å.

18. Use according to one or more of the preceding claims, **characterized in that** the active carbon employed has a raw density in the range from 700 to 975 g/cm³, in particular 750 to 950 g/cm³, preferably 800 to 900 g/cm³, and/or **in that** the active carbon employed has a total porosity of 40 to 70 %, in particular 45 to 65 %, preferably 50 to 60 % and/or **in that** the active carbon employed has a specific total pore volume in the range from 0.1 to 2.5 cm³/g, in particular 0.2 to 2.0 cm³/g, preferably 0.3 to 1.5 cm³/g, particularly preferably 0.4 to 1.0 cm³/g, in particular where the content of pores having pore diameters ≤ 36 Å is at least 65 %, in particular at least 70 %, preferably at least 75 %, and up to 95 %, in particular up to 90 %.

## Revendications

1. Utilisation d'un charbon actif particulaire, en particulier sous forme de grains de charbon actif, de préférence de perles de charbon actif, pour la préparation d'un médicament destiné au traitement de maladies du tractus gastro-intestinal, le charbon actif utilisé pouvant être obtenu par carbonisation puis activation de copolymères styrène/divinylbenzène, le charbon actif utilisé présentant une proportion en volume des micropores ayant un diamètre de pore ≤ 25 Å d'au moins 60 % par rapport au volume total des pores du charbon actif, pour un volume total des pores, selon Gurvich, de 0,50 à 0,90 cm³/g.

2. Utilisation selon la revendication 1 pour préparer un médicament destiné à être utilisé chez un humain ou un animal.

3. Utilisation selon la revendication 1 ou 2, pour préparer un médicament destiné au traitement de maladies inflammatoires du tractus gastro-intestinal.

4. Utilisation selon l'une ou plusieurs des revendications précédentes pour préparer un médicament destiné au traitement de maladies inflammatoires, en particulier d'origine bactérienne ou virale, de l'intestin grêle et/ou du gros intestin, en particulier de l'entérocolite.

5. Utilisation selon l'une ou plusieurs des revendications précédentes pour préparer un médicament destiné au traitement de maladies diarrhéiques (diarrhée).

6. Utilisation selon l'une ou plusieurs des revendications précédentes pour préparer un médicament destiné au traitement d'empoisonnements, en particulier d'empoisonnements alimentaires ou d'empoisonnements après prise de substances toxiques par voie orale.

7. Utilisation selon l'une ou plusieurs des revendications précédentes, **caractérisée en ce que** le charbon actif utilisé comprend des grains de charbon actif discrets, de préférence des perles de charbon actif, ou en est constitué, les grains de charbon actif, de préférence les perles de charbon actif, présentant une pression d'éclatement d'au moins 5 newtons, en particulier comprise dans la plage de 5 newtons à 20 newtons, par granulé ou perle, et/ou que le charbon actif utilisé est un charbon actif sphérique, et/ou que le charbon actif utilisé comprend des grains de charbon actif, de préférence des perles de charbon actif, ayant une granulométrie moyenne comprise dans la plage de 0,01 à 2,0 mm, en particulier de 0,05 à 1,0 mm, de préférence de 0,1 à 1,0 mm.

8. Utilisation selon l'une ou plusieurs des revendications précédentes, **caractérisée en ce que** le charbon utilisé peut être obtenu par une carbonisation, suivie d'une activation, de copolymères de styrène/divinylbenzène sous forme granulaire, en particulier sous forme de sphères, et/ou que la teneur des copolymères styrène/divinylbenzène en divinylbenzène est comprise dans la plage de 1 à 15 % en poids, de préférence de 2 à 10 % en poids, par rapport aux copolymères styrène/divinylbenzène.

9. Utilisation selon l'une ou plusieurs des revendications précédentes, **caractérisée en ce que** le charbon actif utilisé peut être obtenu par carbonisation, suivie d'une activation, de polystyrènes réticulés à du divinylbenzène.

10. Utilisation selon l'une ou plusieurs des revendications précédentes, **caractérisée en ce que** le charbon actif utilisé a une aire spécifique (aire BET) d'au moins 500 m²/g, en particulier d'au moins 750 m²/g, de préférence d'au moins 1000 m²/g, d'une manière particulièrement préférée d'au moins 1200 m²/g, et/ou que le charbon actif utilisé a une aire spécifique (aire BET) de 500 à 2500 m²/g, en particulier de 750 à 2250 m²/g, de préférence de 900 à 2000 m²/g, d'une manière particulièrement préférée de 1000 à 1750 m²/g.

11. Utilisation selon l'une ou plusieurs des revendications précédentes, **caractérisée en ce que** le charbon actif utilisé présente un volume d'adsorption V_{ads} d'au moins 250 cm³/g, en particulier d'au moins 300 cm³/g, de préférence d'au moins 350 cm³/g, d'une manière particulièrement préférée d'au moins 400 cm³/g, et/ou que le charbon actif utilisé présente un volume d'adsorption V_{ads} de 250 à 1000 cm³/g, en particulier de 300 à 900 cm³/g, de préférence de 350 à 750 cm³/g, et/ou que le charbon actif utilisé présente un volume total des pores selon Gurvich de 0,55 à 0,85 cm³/g, de préférence de 0,60 à 0,80 cm³/g, d'une manière particulièrement préférée de 0,65 à 0,80 cm³/g, d'une manière tout particulièrement préférée de 0,70 à 0,75 cm³/g.

12. Utilisation selon l'une ou plusieurs des revendications précédentes, **caractérisée en ce que** le charbon actif utilisé présente, par rapport au volume total des pores, une proportion en volume des micropores ayant un diamètre de pore ≤ 25 Å, d'au moins 65 %, de préférence d'au moins 70 %.

13. Utilisation selon l'une ou plusieurs des revendications précédentes, **caractérisée en ce que** le charbon actif utilisé présente, par rapport au volume total des pores, une proportion en volume des pores formés de micropores ayant un diamètre de pore s 20 Å d'au moins 60 %, en particulier d'au moins 65 %, de préférence d'au moins 70 %, et que le charbon actif utilisé présente, par rapport au volume total des pores, une proportion en volume des micropores formés de pores ayant un diamètre de pore s 25 Å, en particulier ≤ 20 Å, de 60 à 95 %, en particulier de 65 à 90 %, de préférence de 70 à 85 %.

14. Utilisation selon l'une ou plusieurs des revendications précédentes, **caractérisée en ce que** le charbon actif présente un volume, par la méthode au noir de carbone, des pores formés de micropores ayant un diamètre de pores ≤ 25 Å, de préférence ≤ 20 Å, d'au moins 0,40 cm³/g, en particulier d'au moins 0,45 cm³/g, de préférence d'au moins 0,50 cm³/g, et/ou que le charbon actif utilisé présente un volume, par la méthode au noir de carbone, des pores formés de micropores ayant un diamètre de pores s 25 Å, de préférence ≤ 20 Å, de 0,40 à 0,80 cm³/g, en particulier de 0,45 à 0,75 cm³/g, de préférence de 0,50 à 0,6 cm³/g.

15. Utilisation selon l'une ou plusieurs des revendications précédentes, **caractérisée en ce que** le charbon actif utilisé présente, par rapport à l'aire spécifique totale (BET) du charbon actif, une proportion de l'aire spécifique formée des micropores ayant un diamètre de pores ≤ 25 Å, de préférence ≤ 20 Å, d'au moins 70 %, en particulier d'au moins 75 %, de préférence d'au moins 80 %, d'une manière tout particulièrement préférée d'au moins 85 %, et/ou que le charbon actif utilisé présente, par rapport à l'aire spécifique totale (BET) du charbon actif, une proportion de l'aire spécifique des pores formés de micropores ayant un diamètre de pores ≤ 25 Å, de préférence ≤ 20 Å, de 70 à 95 %, en particulier de 75 à 95 %, de préférence de 80 à 90%.

16. Utilisation selon l'une ou plusieurs des revendications précédentes, **caractérisée en ce que** le charbon actif utilisé présente une aire, déterminée par la méthode au noir de carbone, des pores formés de micropores ayant un diamètre de pores ≤ 25 Å, de préférence ≤ 20 Å, d'au moins 400 m²/g, en particulier d'au moins 800 m²/g, de préférence d'au moins 1000 m²/g, d'une manière particulièrement préférée d'au moins 1200 m²/g, et/ou que le charbon actif utilisé présente, par la méthode au noir de carbone, une aire des pores formés de micropores ayant un diamètre de pores s 25 Å, de préférence ≤ 20 Å, de 400 à 1750 m²/g, en particulier de 800 à 1500 m²/g, de préférence de 1000 à 1400 m²/g, d'une manière particulièrement préférée de 1100 à 1300 m²/g.

17. Utilisation selon l'une ou plusieurs des revendications précédentes, **caractérisée en ce que** le charbon actif utilisé présente un diamètre moyen des pores (moyenne du diamètre des pores) d'au plus 35 Å, de préférence d'au plus 30 Å, d'une manière particulièrement préférée d'au plus 25 Å, et/ou que le charbon actif utilisé présente un diamètre moyen des pores (moyenne du diamètre des pores) compris dans la plage de 15 à 35 Å, en particulier de 15 à 30 Å, de préférence de 20 à 25 Å.

18. Utilisation selon l'une ou plusieurs des revendications précédentes, **caractérisée en ce que** le charbon actif utilisé présente une masse volumique apparente comprise dans la plage de 700 à 975 g/cm³, en particulier de 750 à 950 g/cm³, de préférence de 800 à 900 g/cm³, et/ou que le charbon actif utilisé présente une porosité totale de 40 à 70 %, en particulier de 45 à 65 %, de préférence de 50 à 60 %, et/ou que le charbon actif utilisé présente un volume spécifique total des pores compris dans la plage de 0,1 à 2,5 cm³/g, en particulier de 0,2 à 2,0 cm³/g, de préférence de 0,3 à 1,5 cm³/g, d'une manière particulièrement préférée de 0,4 à 1,0 cm³/g, la proportion des pores ayant un diamètre de pore ≤ 36 Å étant en particulier d'au moins 65 %, en particulier d'au moins 70 %, de préférence d'au moins 75 % et allant jusqu'à 95 %, en particulier allant jusqu'à 90 %.
